# NEUE EUROPÄISCHE PATENTSCHRIFT

(11) **EP 2 371 397 B2**
(45) Veröffentlichungstag und Bekanntmachung des Hinweises auf die Entscheidung über den Einspruch: **16.02.2022**
(45) Hinweis auf die Patenterteilung: 25.02.2015
(21) Anmeldenummer: 11159324.0
(22) Anmeldetag: 23.03.2011
(51) Int. Cl.: A61L 2/08, B65B 55/02, B67C 7/00

(54) **Vorrichtung zum Sterilisieren von Behältnissen**
Device for sterilising containers
Dispositif de stérilisation de récipients

(30) Priorität: 23.03.2010 DE 102010012569
(43) Veröffentlichungstag der Anmeldung: 05.10.2011
(73) Patentinhaber: Krones AG, 93073 Neutraubling (DE)
(72) Erfinder: Meinzinger, Rupert, 94356, Kirchroth (DE); Krüger, Dr. Jochen, 93095 Hagelstadt (DE)
(74) Vertreter: Bittner, Bernhard

(56) Entgegenhaltungen:
- EP-A1- 2 103 528
- EP-A1- 2 161 202
- EP-A1- 2 213 578
- WO-A1-2007/140883
- WO-A1-2009/052800
- WO-A1-2011/011079
- WO-A2-2009/095182
- US-A1- 2011 012 032

## Beschreibung

Die vorliegende Erfindung bezieht sich auf eine Vorrichtung und ein Verfahren zum Sterilisieren von Behältnissen. Im Bereich der getränkeherstellenden Industrie ist es seit längerem bekannt, dass zu befüllende Behältnisse und insbesondere Kunststoffbehältnisse vor dem Befüllen sterilisiert werden. Dabei werden im Stand der Technik üblicherweise sterilisierende Chemikalien wie z. B. Peressigsäure oder Wasserstoffperoxid verwendet. Der Nachteil dieser Substanzen besteht darin, dass üblicherweise diese Chemikalien nach der jeweiligen Behandlung mit Sterilwasser aus den Behältnissen ausgespült werden müssen.

Daher sind aus dem Stand der Technik auch diverse Verfahren bekannt, bei denen zur Sterilisation Strahlung verwendet wird.

WO2009095182 beschreibt ein Verfahren und eine Vorrichtung zum Sterilisieren von Flaschen durch innere und äußere Behandlung der Flaschen mit Elektronenstrahlung, wobei die Vorrichtung Außenwände und Einbauten zum Schutz vor Strahlung aufweist.

Die WO 2008/129397 beschreibt ein Sterilisationssystem für PET-Behältnisse, welche Röntgenstrahlung erzeugt, welche wiederum zur Sterilisation der Behältnisse beiträgt.

Aus der DE 10 2008 007 428 A1 sind ein Verfahren und eine Vorrichtung zum Sterilisieren von Packmitteln bekannt. Dabei werden Behältnisse mittels eines Behälterträgers geführt, um 90 ° geschwenkt und über einen Behandlungskopf mit einer Elektronenstrahlenquelle geschoben.

Diese Vorrichtung erfordert jedoch eine relativ aufwändige Umlenkung für die Behältnisse.

Weiterhin wird im Falle der DE 10 2008 007 428 A1 auch eine Sterilisation der Behältnisaußenwand vorgenommen.

Die EP 1 982 920 A1 beschreibt ebenfalls eine Vorrichtung zum Sterilisieren von Behältnissen. Dabei ist ein Behandlungskopf vorgesehen, der durch die Mündung der Behältnisse in dieses führbar ist, wobei der Behandlungskopf ein Außengehäuse und ein Innengehäuse aufweist und zwischen dem Außengehäuse und dem Innengehäuse ein gasförmiges Medium zur Kühlung führbar ist. Der Gegenstand der EP 1 982 920 A1 wird hiermit vollumfänglich durch Bezugnahme zum Gegenstand auch der vorliegenden Anmeldung gemacht.

Die DE 10 2008 025 868 A1 beschreibt eine Vorrichtung zum Sterilisieren von Behältnissen mittels Ladungsträgern. Dabei ist eine Kühleinrichtung vorgesehen, um das Austrittsfenster von Strahlfingern, aus denen Elektronenstrahlung austritt, zu kühlen. Der Gegenstand der DE 10 2008 025 868 A1 wird hiermit vollumfänglich durch Bezugnahme zum Gegenstand auch der vorliegenden Anmeldung gemacht.

Die bisher noch unveröffentlichte DE 10 2008 045 187.8 beschreibt ebenfalls ein Verfahren zur Elektronenstrahlsterilisation für Behältnisse. Dabei wird eine Bewegungsgeschwindigkeit eines im Inneren der Behältnisse befindlichen Behandlungskopfes während eines vorgegebenen Zeitraums variiert und in Abhängigkeit von einem Innenprofil des Behältnisses gesteuert. Der Gegenstand der DE 10 2008 045 187.8 wird hiermit vollumfänglich durch Bezugnahme zum Gegenstand auch der vorliegenden Anmeldung gemacht.

Der vorliegenden Erfindung liegt daher die Aufgabe zugrunde, eine Vorrichtung zum Sterilisieren von Behältnissen mittels Elektronenstrahlen zur Verfügung zu stellen, welche eine verbesserte Gesamtsterilisation der zu sterilisierenden Behältnisse erlaubt.

Eine erfindungsgemäße Vorrichtung zum Sterilisieren von Behältnissen wird durch Anspruch 1 definiert.

Vorteilhaft erstreckt sich die Längsrichtung des stangenartigen Elements auch im Wesentlichen senkrecht zu einer Bewegungsebene, in der (unter Außerachtlassung einer Hubbewegung während des Sterilisationsprozesses) die Behältnisse bewegt werden. Unter im Wesentlichen senkrecht wird dabei verstanden, dass ein Winkel, unter dem die Längsrichtung auf die besagte Ebene trifft, zwischen 75° und 105°, bevorzugt zwischen 80° und 100° und besonders bevorzugt zwischen 85° und 95° liegt. Vorteilhaft ist die Längsrichtung des stangenartigen Elements stets im Wesentlichen parallel zu der Längsrichtung der zu sterilisierenden Behältnisse.

Vorteilhaft sind die zweiten Strahlungserzeugungseinrichtungen stets außerhalb der zu sterilisierenden Behältnisse angeordnet, beispielsweise seitlich entlang eines Transportpfades der Behältnisse. Unter Strahlung wird im Rahmen dieser Beschreibung nicht nur Strahlung im engeren Sinne wie Licht, UV- oder Röntgenstrahlung verstanden sondern auch Teilchenstrahlung, wie insbesondere Elektronenstrahlung. Damit stellt auch eine erzeugte Elektronenwolke Strahlung im Sinne dieser Beschreibung dar.

Bei einer weiteren vorteilhaften Ausführungsform sind die ersten Sterilisationseinrichtungen beweglich angeordnet. Dies bedeutet, dass sie sich insbesondere in Richtung des Transportpfades der Behältnisse und besonders bevorzugt mit den Behältnissen mit bewegen. Auf diese Weise ist ein im Wesentlichen kontinuierlicher Betrieb der Vorrichtung bzw. auch der Transporteinrichtung möglich.

Bei einer weiteren vorteilhaften Ausführungsform sind die Sterilisationseinrichtungen an der Transporteinrichtung angeordnet und bewegen sich daher mit dieser mit.

Vorteilhaft weist die Vorrichtung eine Ausblaseinheit auf, welche die Behältnisse nach der Sterilisation mit dem Sterilisationselement mit einem gasförmigen Medium und insbesondere mit Sterilluft beaufschlagt. Auf diese Weise ist es möglich, die bei der Elektronenbestrahlung entstehende Menge an Ozon und Stickoxiden wieder aus dem Behältnis zu entfernen. Diese Ausblaseinheit kann dabei an den Sterilisationselementen vorgesehen sein. Dabei wäre es auch möglich, dass diejenige Kühlluft, welche zum Kühlen des Austrittsfensters der Strahlungselemente verwendet wird, durch entsprechende Umlenkung auch zum Ausblasen der Behältnisse verwendet wird. Vorzugsweise kann dabei an den Sterilisationselementen eine Umlenkeinrichtung vorgesehen sein, welche Luft auf die Innenfläche der Behältnisse richtet.

Durch die erfindungsgemäße Vorgehensweise können die fertig hergestellten bzw. geblasenen Behältnisse auf einer Transporteinrichtung wie einem Behandlungskarussell der Elektronenstrahlung ausgesetzt werden, um auf diese Weise die gesamte Innen- und Außenfläche der Behältnisse mit einer ausreichenden Energiedosis zu bestrahlen und um sie anschließend steril an einen Füller zu übergeben.

Die Vorrichtung weist eine Abschirmungseinrichtung auf, welche die sterilisierende Strahlung bzw. die sich aus dieser sterilisierenden Strahlung ergebende Strahlung wenigstens teilweise gegenüber der Umgebung abschirmt. Bei dieser sich aus der sterilisierenden Strahlung ergebenen Strahlung handelt es sich insbesondere um Röntgenstrahlung. Dabei ist es vorteilhaft, wenn die Umgebung und damit auch die Bediener der Behandlungsmaschine vor dieser Strahlung geschützt werden. Vorteilhaft kombiniert daher eine entsprechende Ausführungsform der erfindungsgemäßen Vorrichtung die Innensterilisation der Behältnisse mit einer Außensterilisation der Behältnisse und auch einer Abschirmung der Vorrichtung selbst, insbesondere gegen austretende Röntgenstrahlen.

Vorteilhaft ist die zweite Sterilisationseinrichtung in der Transportrichtung der Behältnisse vor oder nach der ersten Sterilisationseinrichtung angeordnet. Vorteilhaft ist die zweite Sterilisationseinrichtung vor den ersten Sterilisationseinrichtungen angeordnet, so dass zunächst eine Sterilisation der Außenfläche der Behältnisse und anschließend eine Sterilisation der Innenfläche der Behältnisse vorgenommen wird. Daneben kann auch eine entsprechende Sterilisation von Behandlungselementen, etwa von Greifelementen, welche die Behältnisse halten, vorgenommen werden. Besonders vorteilhaft ist die zweite Sterilisationseinrichtung so angeordnet, dass eine davon getrennte Sterilisationseinrichtung, die Transportelemente sterilisiert, nicht notwendig ist. Es wäre jedoch auch denkbar, dass eine Außensterilisation und eine Innensterilisation der Behältnisse gleichzeitig stattfinden, bzw. die zweiten Sterilisationseinrichtungen in einem Bereich der Vorrichtung angeordnet sind, in dem auch eine Innensterilisation der Behältnisse stattfindet. Bei den Behältnissen handelt es sich insbesondere um Kunststoffbehältnisse, für welche sich die hier vorgeschlagene Art der Sterilisierung besonders eignet.

Die sterilisierende Strahlung ist eine Elektronenstrahlung.

Um die Innenfläche der Behältnisse zu bestrahlen, wird ein Elektronenstrahlfinger durch die Mündung der Behältnisse in deren Innenraum eingeführt. Vorteilhaft weist die Sterilisationseinrichtung eine kompakte Elektronenstrahleinheit auf, welche wiederum einen Strahlfinger aufweist, der so dimensioniert ist, dass er in die Behältnisse eintauchen kann, um Elektronenwolken mit möglichst geringer Energie auf die innere Oberfläche der Behältnisse anzuwenden.

Auf diese Weise ist es möglich, eine Sterilisation der Innenfläche der Behältnisse mit einem Volumen bis zu 1,5 I innerhalb von weniger Sekunden und insbesondere innerhalb von 2 Sekunden zu erreichen. Die Behandlungszeit von Behältnissen mit einem Volumen, welches unter 0,5 I liegt, beträgt vorteilhaft weniger als 1 Sekunde.

Bei einer weiteren vorteilhaften Ausführungsform weist die Vorrichtung einen drehbaren Träger auf, an dem die Transportelemente zum Transportieren der Behältnisse angeordnet sind. Vorteilhaft sind auch die Sterilisationselemente an diesem Träger bzw. an der Transporteinrichtung angeordnet. Dabei erstrecken sich die Längsrichtungen der Sterilisationselemente vorteilhaft im Wesentlichen parallel zu einer Drehachse, um welche der besagte Träger drehbar angeordnet ist. Vorteilhaft ist eine Hubeinrichtung vorgesehen, welche die Behältnisse jeweils hebt, so dass durch diese Hubbewegung die Sterilisationselemente in das Innere der jeweiligen Behältnisse eintauchen können.

Vorteilhaft weist die Transporteinrichtung auch Greifelemente auf, welche die Behältnisse - insbesondere an bzw. unter ihren Mündungen - greifen, so dass die Sterilisationselemente in das Innere der Behältnisse eintreten können. Es wäre jedoch auch ein Transport der Behältnisse an ihren Böden oder auch an ihren Seitenwandungen denkbar. Vorteilhaft werden die Behältnisse von der Transporteinrichtung vereinzelt geführt.

Es ist weiterhin möglich, in Abhängigkeit von der Flaschenleistung an einer bestimmten Maschine eine jeweils ausreichende Zahl an Sterilisationselementen bzw. Strahlfingern insbesondere auf einem Rundläufer anzuordnen. So ist es beispielsweise möglich, dass ein Rundläufer mit 24 Strahlfingern gleichzeitig etwa 20 Flaschen innen behandelt. Die übrigen 4 Stationen befinden sich im Leerlauf zwischen den beiden Behältnisübergabesternen. Auf diese Weise ist es möglich, ca. 36.000 Flaschen pro Stunde mit einem Volumen von 1,5 I ausreichend zu sterilisieren. Für kleinere Behältnisse können in der gleichen Anordnung entsprechend mehr Flaschen pro Stunde innen sterilisiert werden, da sich die Sterilisationsprozesse entsprechend verkürzen.

Wie oben erwähnt, sind vorteilhaft auch bei einer Rundläuferanordnung die Sterilisationselemente bzw. Strahlfinger starr an dem Behandlungskarussell bzw. der Transporteinrichtung befestigt. Die jeweiligen Transformatoren bzw. Netzgeräte für die Elektronenstrahlerzeuger können auf dem Karussell mitlaufen, wodurch die Zuführung der Hochspannung (ca. 150 kV) vereinfacht wird.

Bei einer weiteren vorteilhaften Ausführungsform ist die zweite Strahlungserzeugungseinrichtung stationär gegenüber dem Transportpfad der Behältnisse angeordnet. Auf diese Weise werden die einzelnen Behältnisse an dieser zweiten Strahlungserzeugungseinrichtung vorbeigeführt. In vielen Anwendungsbereichen ist es nicht ausreichend, die Behältnisse nur innen zu behandeln. Wenn die Behältnisse in den Sterilbereich einer Fülleinrichtung übergeben werden sollen, ist es auch erforderlich, dass sie an der Außenseite eine gewisse Sterilität aufweisen. Hierzu werden die Behältnisse auch auf der Außenseite mit Strahlung insbesondere mit Elektronenstrahlen beaufschlagt.

Vorteilhaft ist die zweite Strahlungserzeugung jedoch in anderer Weise gestaltet als die erste Strahlungserzeugungseinrichtung. Während bei der ersten Strahlungserzeugungseinrichtung die Strahlung auf eine kleine Fläche konzentriert ist handelt es sich vorteilhaft bei der zweiten Strahlungserzeugungseinrichtung um eine Strahlquelle, deren Austrittsfenster gleichmäßig auf eine große Fläche verteilt ist insbesondere die Außenfläche der zu sterilisierenden Behältnisse. Die Behältnisse werden dann im Vorbeifahren an dem Karussell oder auch an einem der Übergabesterne von außen bestrahlt. Hierzu kann beispielsweise ein kompakter Flächenstrahler Anwendung finden. Diese zweiten Strahlungseinrichtungen werden an einer geeigneten Stelle in der Maschine angeordnet und vorzugsweise derart ausgerichtet, dass die Behältnisse mit einer möglichst großen Fläche durch die Elektronenwolke dieser zweiten Strahlungseinrichtung treten.

Vorteilhaft weist die Vorrichtung eine Dreheinrichtung auf, welche die Behältnisse wenigstens abschnittsweise um ihre Längsrichtung dreht. Mit anderen Worten werden die Behältnisse während dieser Sterilisation durch die zweite Sterilisationseinrichtung gedreht um rund herum bestrahlt zu werden. Auch wäre es möglich, eine weitere zweite Strahlungseinrichtung an einer anderen Stelle der Vorrichtung anzuordnen, so dass die Behältnisse nacheinander oder gleichzeitig von zwei Seiten bestrahlt werden können. Weiterhin könnte noch eine dritte Strahlungseinrichtung vorgesehen sein, um beispielsweise auch die Unterseite oder den Gewindebereich der Behältnisse zu sterilisieren. Daneben könnten auch Sterilisationseinrichtungen vorgesehen sein, welche Behältnisverschlüsse, welche später auf die Behältnisse geschraubt werden, ebenfalls sterilisieren.

Bei einer weiteren vorteilhaften Ausführungsform weist die Abschirmeinrichtung ein Gehäuse auf, welches die Transporteinrichtung wenigstens teilweise umgibt. Wie oben erwähnt, erzeugen Elektronenstrahlen beim Abbremsen Röntgenstrahlung. Die Energie und Intensität dieser Röntgenstrahlung hängt dabei von der Beschleunigungsspannung der Emitter und von dem bestrahlten Material ab. Hierzu zählt nicht nur das bestrahlte PET sondern jedes Material, welches von der Elektronenstrahlung getroffen wird. Dabei kann es sich insbesondere auch um das Metall der Behältnishalterungen und nicht zuletzt auch um die Umgebungsluft der Elektronenwolke handeln.

Die Beschleunigungsspannung der jeweiligen Emitter d. h. sowohl für die Innen- als auch für die Außenbehandlung liegt bei maximal 150 kV. Die intensivste Röntgenstrahlung entsteht, wenn diese Elektronen auf ein Material mit hoher Kernladungszahl treffen. Dabei entsteht jedoch die Röntgenstrahlung nur im Bereich des Behandlungskarussells d. h. in der unmittelbaren Nähe der jeweiligen Emitter. Diese Strahlung darf nicht in die Umgebung der Maschine gelangen. Um Röntgenstrahlung dieser Erzeugungsenergie ausreichend nach außen hin abzuschirmen und damit einen Schutz der Bediener und der Umgebung zu gewährleisten, ist für einen geradlinigen Weg nach außen eine Wand aus Blei in einer Stärke von ca. 5-6 mm notwendig. Auch tritt eine ausreichende Abschirmung der Strahlungseinrichtung auf, wenn sie zwei- bis dreimal an der Oberfläche reflektiert wird.

Allerdings gelten für die Reflektion von Röntgenstrahlung nicht die Regeln der geometrischen Optik sondern die Strahlung kann, nachdem sie auf eine Oberfläche auftrifft in eine beliebige Richtung gestreut werden. Anstelle von Blei wäre es auch möglich, andere Materialien zu verwenden, wobei dieses dann jedoch eine äquivalente Dicke aufweisen muss. So ist es beispielsweise möglich anstelle von Blei einen Stahlmantel mit einer Dicke im Bereich von 80 mm zu wählen.

Zusätzlich zu dem Gehäuse kann im Inneren ein Wandungslabyrinth vorgesehen sein, welches an jedem Punkt außerhalb der Maschine gewährleistet, dass die im Inneren der Maschine entstehende Strahlung geradlinig durch mindestens 80 mm Stahl abgeschirmt wird oder dass der Strahl mindestens zweimal, bevorzugt mindestens dreimal reflektiert wird, bevor er ins Freie gelangt.

Vorteilhaft weist das Gehäuse wenigstens eine Zuführöffnung auf, um dem Gehäuse die Behältnisse zuzuführen. Daneben weist das Gehäuse vorteilhaft auch eine Öffnung auf, um die Behältnisse aus dem Gehäuse abzuführen. Dabei ist es möglich, dass diese beiden Öffnungen ineinander übergehen, so dass die Größe der gesamten Öffnung minimiert werden kann. Vorteilhaft weist das Gehäuse abgesehen von diesen Öffnungen keine weiteren Öffnungen auf. Dabei kann jedoch bevorzugt vorgesehen sein, dass das Gehäuse wenigstens eine bewegliche Außenwand aufweist, so dass es, insbesondere zu Wartungszwecken, geöffnet werden kann. Bevorzugt kann das Gehäuse derart geöffnet werden, dass sämtliche im Inneren des Gehäuses liegenden Anlagenteile für den Benutzer zugänglich sind.

Vorteilhaft kann durch die besagten Öffnungen, durch welche die Behältnisse zu- bzw. abgeführt werden, von außen keiner der Emitter (bzw. dessen eigentliche Strahlungsquelle) entlang eines geradlinigen Beobachtungspfades gesehen werden. Bevorzugt ist auch die Transporteinrichtung, an der die ersten Sterilisationseinrichtungen angeordnet sind, durch die besagte Öffnung nicht sichtbar.

Vorteilhaft sind im Inneren des Gehäuses Innenwandungen angeordnet, welche daher bewirken, dass im Inneren des Gehäuses entstehende Strahlung wenigstens einmal und bevorzugt wenigstens zweimal und besonderes bevorzugt wenigstens dreimal reflektiert wird, bevor sie zu der besagten Zuführöffnung gelangt. Diese Wandungen bilden bevorzugt gemeinsam mit der Gehäuseaußenwand die Abschirmungseinrichtung. Vorteilhaft bilden die besagten Innenwandungen innerhalb des Gehäuses ein Labyrinth, durch welches die Behältnisse geführt werden.

Die vorliegende Erfindung ist weiterhin auf ein Verfahren zum Sterilisieren von Behältnissen gerichtet, das durch Anspruch 5 definiert wird.

Es wird daher auch verfahrensseitig vorgeschlagen, dass eine Sterilisation sowohl der Innenals auch der Außenflächen der Behältnisse vorgenommen wird.

Bei einem weiteren vorteilhaften Verfahren werden die Sterilisationselemente in der Transportrichtung der Behältnisse bewegt. Vorteilhaft werden die Sterilisationselemente mit den Behältnissen mitbewegt. Vorteilhaft weist die Vorrichtung eine Vielzahl von Halteeinrichtungen für die Behältnisse auf und jede Halteeinrichtung ist vorteilhaft einem Sterilisationselement zugeordnet.

So ist es möglich, dass diese Halteeinrichtungen und die Sterilisationselemente auf einen gemeinsamen Träger angeordnet sind und sich so gemeinsam bewegen. Vorteilhaft werden die Halteeinrichtungen in einer senkrecht zu der Transportrichtung stehenden Richtung bewegt, um die Behältnisse in einer Längsrichtung der Sterilisationselemente zu bewegen. Auf diese Weise ist es möglich, durch eine Bewegung der Behältnisse die Sterilisationselemente in die Behältnisse einzuführen, so dass die Innenwandung der Behältnisse (von innen) sterilisiert werden kann.

Vorteilhaft sind die Sterilisationselemente fest in einer zu dem Transportpfad senkrechten Richtung angeordnet.

Bei einem weiteren bevorzugten Verfahren weist ein Transportpfad der Behältnisse innerhalb der Vorrichtung wenigstens zwei unterschiedliche Krümmungen auf. Auf diese Weise kann der Transportpfad zwischen Wandungen innerhalb des Behältnisses durchgeführt werden, so dass entstehende Strahlung, wie oben erwähnt, wenigstens zweimal bevorzugt wenigstens dreimal reflektiert wird um aus dem Gehäuse heraus zu gelangen.

Genauer gesagt werden die Behältnisse an diesen einzelnen Wänden vorbei bzw. um diese Wände herumgeführt.

Weitere Vorteile und Ausführungsformen ergeben sich aus den beigefügten Figuren.

Darin zeigen:
Fig. 1 : eine Übersicht über eine Anlage zum Behandeln von Behältnissen;
Fig. 2 : eine Darstellung eines Anlagenteils zur Innensterilisation von Behältnissen;
Fig. 3 : eine Darstellung zur Veranschaulichung der Außensterilisation von Behältnissen;
Fig. 4 : eine erfindungsgemäße Vorrichtung in einer ersten Ausführungsform;
Fig. 5 : eine erfindungsgemäße Vorrichtung in einer zweiten Ausführungsform;
Fig. 6 : eine nicht erfindungsgemäße Vorrichtung in einer dritten Ausführungsform; und
Fig. 7 : eine nicht erfindungsgemäße Vorrichtung in einer vierten Ausführungsform.

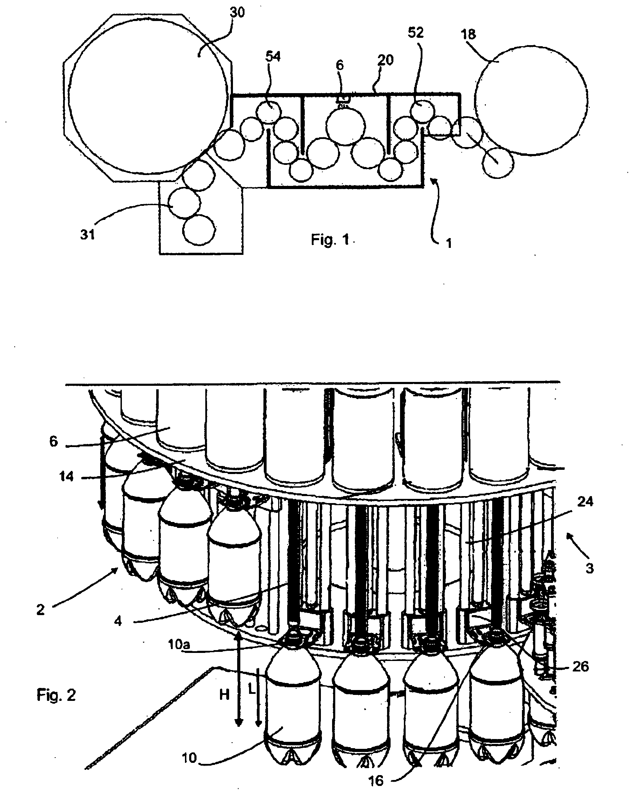

Fig. 1 zeigt eine grob schematische Darstellung einer Vorrichtung 1 zum Behandeln von Behältnissen. Diese Anlage weist dabei eine Umformungseinheit 18 auf, welche Kunststoffvorformlinge durch einen Blasformungsvorgang zu Kunststoffbehältnissen umformt.

Dabei werden Kunststoffvorformlinge zunächst über einen (nicht gezeigten) Ofen erwärmt und anschließend zu Kunststoffbehältnissen blasgeformt. Diese hergestellten Kunststoffbehältnisse werden über eine Vielzahl von Transporteinrichtungen wie Transporträder oder Transportsterne 52 zu einer Sterilisationseinrichtung 6 gefördert, welche die Behältnisse mittels Strahlung sterilisiert. Nach diesem Sterilisationsvorgang werden die Behältnisse wiederum über eine Vielzahl von Transporträdern 54 transportiert und an eine Fülleinrichtung 30 übergeben. An diese Fülleinrichtung 30 schließt sich noch ein Verschließer 31 an, der die Behältnisse mit bevorzugt sterilen Verschlüssen verschließt.

Das Bezugszeichen 20 kennzeichnet in seiner Gesamtheit eine Abschirmeinrichtung, welche die bei der Sterilisation entstehende Strahlung blockiert. Man erkennt, dass hier die einzelnen Transportsterne 52, 54 die Behältnisse entlang eines mehrfach geschwungenen Pfades P führen, um so zu verhindern, dass Strahlung unmittelbar aus der Abschirmeinrichtung 20 austreten kann. Das Bezugszeichen 21 bezieht sich auf ein Gehäuse, welches Bestandteil der Abschirmeinrichtung 20 ist.

Fig. 2 zeigt eine perspektivische Darstellung einer Sterilisationseinrichtung zum Sterilisieren von Behältnissen. Dabei werden die Behältnisse 10 mittels einer in ihrer Gesamtheit mit 2 bezeichneten Transporteinrichtung entlang eines im Wesentlichen kreisförmigen Pfades geführt. Die Transporteinrichtung 2 weist einen Träger 14 auf, der drehbar um eine (nicht gezeigte) Drehachse angeordnet ist. Der Träger 14 ist dabei Teil der Abschirmeinrichtung 20. Der Spalt zwischen dem rotierenden Träger 14 und dem feststehenden Teil der Abschirmeinrichtung, einem (nicht gezeigten) Isolatordach, wird durch einen (ebenso nicht gezeigten) Ringkanal abgedichtet, in dem durch geeignete Einbauten eine Labyrinthstruktur aufgebaut ist. Dadurch kann durch diesen Spalt keine Strahlung direkt austreten. Bevorzugt wird aus dem Ringkanal Gasatmosphäre abgesaugt. An dem Träger 14 ist weiterhin eine Vielzahl von ersten Sterilisationseinrichtungen 3 angeordnet, wobei diese ersten Sterilisationseinrichtungen 3 jeweils Strahlungserzeugungseinrichtungen 6 aufweisen sowie Strahlungselemente 4. Diese Strahlungselemente 4 sind hier als stab- oder fingerförmige Elemente ausgebildet, welche in die Behältnisse 10 bzw. deren Mündungen 10a einführbar sind.

Zu diesem Zweck weist die Vorrichtung eine Vielzahl von Greifelementen 16 auf, welche die Behältnisse an ihrer Mündung bzw. genauer unterhalb ihres Tragrings halten. Zum Einführen der Strahlungselemente 4 werden die Behältnisse um den Hub H angehoben, so dass die Strahlungselemente in das Innere der Behältnisse eindringen. Dabei können die Behältnisse 10 bevorzugt durch (nicht gezeigte), insbesondere mit den Greifelementen hebbare, Führungen seitlich abgestützt werden, so dass die Behältnisse stets exakt zu den Strahlungselementen ausgerichtet sind. Aus einem unteren Bereich der Strahlungselemente tritt Elektronenstrahlung aus und trifft auf die Innenwandung der Behältnisse, welche auf diese Weise sterilisiert werden kann. Das Bezugszeichen 24 bezieht sich auf Führungselemente, an denen Schlitten 26 angeordnet sind und an den Schlitten 26 sind wiederum die Greifelemente 16 vorgesehen, welche auf diese Weise die Behältnisse heben und senken. Die Strahlungserzeugungseinrichtungen sind bevorzugt fest an dem Träger 14 angeordnet.

Auf diese Weise ist eine gleichmäßige Behandlung der inneren Oberfläche der Behältnisse 10 möglich, wobei bevorzugt hierzu eine Strahlendosis in einem Bereich 12-15 kGy verwendet wird. Nachdem die Behältnisse 10 während eines Umlaufs einmal über das Strahlungselement 10 hoch und wieder nach unten bewegt wurden, werden sie durch einen weiteren Übergabestern wieder von den in ihrer Gesamtheit mit 2 bezeichneten Transporteinrichtung genommen. Die Hubbewegung der Greifelemente kann dabei über (nicht gezeigte) insbesondere feststehende Führungskurven erreicht werden. Es wäre jedoch auch möglich, dass die Hubbewegungen der einzelnen Greifelemente unabhängig voneinander steuerbar sind und diese Hubbewegungen auch während des jeweiligen Sterilisationsprozesses individuell gesteuert werden, beispielsweise durch den Einsatz von Servomotoren, Linearmotoren oder Ähnlichem. Auch eine pneumatische Ansteuerung ist möglich.

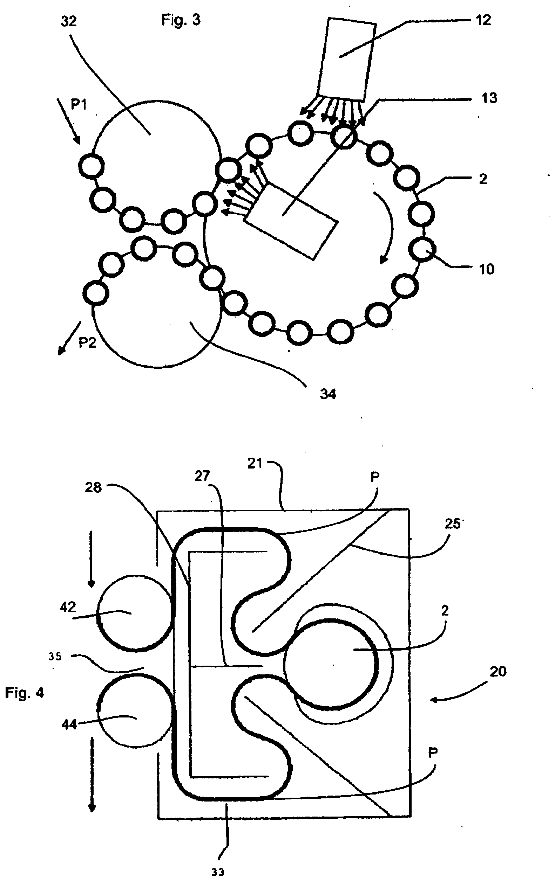

Fig. 3 zeigt eine weitere Darstellung zur Veranschaulichung der Erfindung. Auch hier ist wieder die Transporteinrichtung 2 vorgesehen, mit der eine Vielzahl von Behältnissen 10 geführt wird. Die erste Strahlungseinrichtung 3 ist hier jedoch nicht dargestellt. Bei der in Fig. 3 gezeigten Ausführungsform erfolgt vor oder während der Innenbehandlung der Behältnisse auch eine Bestrahlung der Außenoberfläche der Behältnisse 10. Zu diesem Zweck sind zwei zweite Sterilisationseinrichtungen 12 und 13 vorgesehen, die hier stationär angeordnet sind und den Außenbereich der Behältnisse ebenfalls mit Elektronenstrahlen bestrahlen. Das Bezugszeichen 32 bezieht sich auf ein Zuführrad, welches der Transporteinrichtung 2 die Behältnisse zuführt (Pfeil P1) und das Bezugszeichen 34 ein Abführrad, welche die nunmehr bereits sterilisierten Behältnisse von der Transporteinrichtung 2 abführt (Pfeil P2).

Fig. 4 zeigt eine Darstellung einer möglichen erfindungsgemäßen Anordnung. Dabei ist hier insbesondere eine Abschirmungseinheit 20 vorgesehen, welche hier als ein Gehäuse mit einer Vielzahl von Innenwandungen 25, 27, 28 ausgeführt ist.

Bei der in Fig. 4 gezeigten Ausführungsform werden die Behältnisse von den Transportstern 42 an eine Transportkette 33 (schematisch dargestellt) übergeben. Diese Transportkette 33 bringt die Behältnisse durch ein Schleusenlabyrinth, welches durch die Vielzahl der (Innen)Wände 25, 27, 28 gebildet wird. Auf diese Weise wird erreicht, dass im Inneren des Gehäuses 20 entstehende Strahlung nicht nach außen dringt oder nur in stark abgeschwächter Form. Durch die Anordnung der jeweiligen (Innen)Wände 25, 27, 28 wird erreicht, dass die Strahlung wenigstens dreimal reflektiert wird, bevor sie durch die Öffnung 35 nach außen treten kann. Die Öffnung 35 dient hier sowohl zum Zuführen als auch zum Abführen der Behältnisse. Das Bezugszeichen P kennzeichnet den Transportpfad der Behältnisse im Inneren der Abschirmeinrichtung 20 und vor der Transporteinrichtung 2.

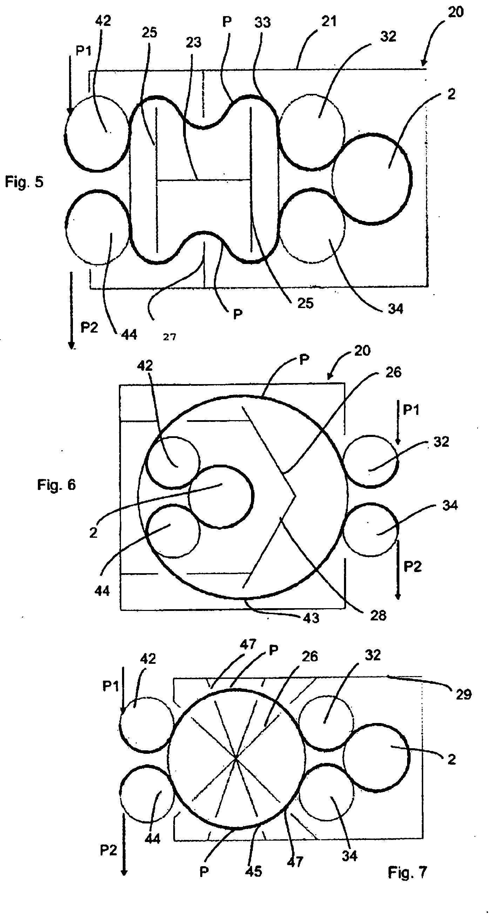

Fig. 5 zeigt eine weitere Ausführungsform einer erfindungsgemäßen Anordnung. Hier werden die Behältnisse ebenfalls wieder von dem Einlaufrad 42 an eine Transportkette 33 übergeben. Diese Transportkette weist dabei bevorzugt eine Vielzahl von Halteeinrichtungen zum Halten der einzelnen Behältnisse auf. Die Transportkette 33 führt die Behältnisse auf einem gekrümmten Pfad durch das Gehäuse so dass durch die einzelnen Innenwände 23, 25, 27 eine ausreichende Abschirmung der entstehenden Röntgenstrahlung erreicht werden kann.

Auch hier werden die Behältnisse durch ein Schleusenlabyrinth, welches durch die Vielzahl von Wänden 23, 25, 27 gebildet wird an ein Zuführrad 32 übergeben, welches die Behältnisse wiederum an die Transporteinrichtung 2 übergibt. Nach der Sterilisation werden die Behältnisse wieder von dem Abführrad 34 entlang des Transportpfades P aus dem Gehäuse 20 ausgeführt. Daneben läuft die Transportkette hier um die Wände 25 und 23 geschlossen um, führt jedoch nicht unmittelbar zu der Transporteinrichtung 2.

Die einzelnen Wandungen haben bei den in den Fig. 4-7 ausgeführten Ausführungsformen jeweils eine Wandstärke von ca. 5 mm und sind vorzugsweise aus Blei hergestellt, bzw. sind mit einer entsprechend starken Bleibeschichtung oder einem Bleibelag versehen. Es wären jedoch auch andere Materialien denkbar, wobei hier die Wandungsstärke gegebenenfalls modifiziert ist um den gleichen Abschirmeffekt zu erreichen.

Auch bei der in Fig. 6 gezeigten Ausführungsform befindet sich die Transporteinrichtung 2 vollständig im Inneren eines Transportkarussells 43. An dieses Transportkarussell 43 werden die Behältnisse übergeben und von diesem Transportkarussell 43 wiederum über das Zuführrad 42 in die Transporteinrichtung 2 und von dort über das Abführrad 44 wieder zurück an das Transportkarussell 43 übergeben. Auch hier sind im Inneren des Gehäuses eine Vielzahl von Wandungen 26, 28 vorgesehen. Auch hier laufen die Behältnisse nach der Behandlung über einen Transferstern 34 zu einer Fülleinrichtung.

Fig. 7 zeigt eine Ausführungsform, bei der die Anordnung lediglich aus Karussells und Sternen aufgebaut ist. Die Behältnisse werden hier zunächst über ein Einführrad 42 entlang des Pfades P1 an ein Transportkarussell 45 übergeben. Dieses wiederum übergibt die Behältnisse ähnlich wie oben an das Zuführrad 32 und auf diese Weise letztlich an die Transporteinrichtung 2. Von dort gelangen die Behältnisse über das Ausführrad 34 wieder zu dem Transportkarussell 45 zu dem Ausführrad 44. Die Wände sind hier derart unterbrochen bzw. weisen Schlitze 47 auf, so dass die Behältnisse durch diese Schlitze bzw. Öffnungen geführt werden. Ein lineares Austreten der unreflektierten Strahlung wird auf diese Weise verhindert. Auch bei der in Fig. 7 gezeigten Ausführungsform könnte die Röntgenstrahlung nur über mehrfache Reflektionen an einer oder mehreren Wänden oder an einer Gehäusewand 29 aus in ihrer Gesamtheit mit 20 bezeichneten Abschirmeinrichtung austreten.

Bei sämtlichen der gezeigten Ausführungsformen ist daher als Abschirmeinrichtung ein Gehäuse vorgesehen, in dem vorteilhaft die Transporteinrichtung 2 in ihrer Gesamtheit untergebracht ist. Vorteilhaft ist auch eine Öffnung 35 dieses Gehäuses 20 von der Transporteinrichtung 2, welche die Behältnisse während deren Innensterilisation transportiert, beabstandet.

Daneben ist vorteilhaft innerhalb der Abschirmeinrichtung 20 neben der Transporteinrichtung noch wenigstens teilweise eine weitere Transporteinheit 33 angeordnet, welche die Behältnisse 10 der Transporteinrichtung 2 zuführt oder von der Transporteinrichtung 2 die Behältnisse abführt. Vorzugsweise ist auch diese weitere Transporteinheit 33 vollständig innerhalb der Abschirmeinrichtung 20 angeordnet, d.h. die Behältnisse 20 werden von dieser weiteren Transporteinheit 33 ausschließlich innerhalb der Abschirmeinrichtung 20 transportiert. Vorteilhaft weist daher die Abschirmeinrichtung auch eine Vielzahl von Innenwänden auf, welche zur Abschirmung der entstehenden Strahlung dienen. Zwischen diesen Innenwandung sind dabei vorteilhaft jeweils Lücken vorgesehen, durch welche die Behältnisse hindurch geführt werden können.

### Bezugszeichenliste

- 1: Vorrichtung
- 2: Transporteinrichtung
- 3: erste Sterilisationseinrichtungen
- 4: Strahlungselemente
- 6: Strahlungserzeugungseinrichtung
- 10: Behältnisse
- 10a: Mündungen
- 12: zweite Sterilisationseinrichtung
- 13: zweite Sterilisationseinrichtung
- 14: Träger
- 16: Greifelemente
- 18: Umformungseinheit
- 20: Abschirmeinrichtung, Gehäuse
- 21: Gehäuse
- 23: Wand
- 24: Führungselemente
- 25: Wand
- 26: Schlitten
- 27: Innenwandung
- 28: Innenwandung
- 29: Gehäusewand
- 30: Fülleinrichtung
- 31: Verschliesser
- 32: Zuführrad, Transportstern
- 33: Transportkette, weitere Transporteinheit
- 34: Abführrad
- 35: Öffnung
- 42: Einlaufrad
- 43: Transportkarussell
- 44: Auslaufrad, Transferstern
- 45: Transportkarussell
- 47: Schlitze
- 52, 54: Transportsterne
- P: Transportpfad
- H: Hub
- L: Längsrichtung

## Patentansprüche

1. Vorrichtung (1) zum Sterilisieren von Behältnissen (10) mit einer Transporteinrichtung (2), welche die Behältnisse (10) entlang eines vorgegebenen Transportpfades transportiert, mit einer Vielzahl von Sterilisationseinrichtungen (3) zum Sterilisieren wenigstens eines Bereiches der Innenwandung der Behältnisse, wobei die ersten Sterilisationseinrichtungen jeweils ein stangenartiges als Strahlungselement ausgebildetes Sterilisationselement (4) aufweisen dessen Längsrichtung (L) sich im Wesentlichen senkrecht zu dem Transportpfad der Behältnisse und stets parallel zur Längsrichtung der zu sterilisierenden Behältnisse erstreckt, und welches durch eine Relativbewegung des Behältnisses (10) gegenüber dem Strahlungselement (4) durch eine Mündung (10a) der zu sterilisierenden Behältnisse (10) hindurch in das Innere der zu sterilisierenden Behältnisse (10) einführbar ist, sowie eine einen Bestandteil der ersten Strahlungseinrichtung (3) bildende Strahlungserzeugungseinrichtung (6), welche eine Elektronenstrahlung erzeugt, wobei die Vorrichtung (1) wenigstens eine zweite Sterilisationseinrichtung (12), geeignet und bestimmt zur Sterilisation wenigstens einer Außenwandung der Behältnisse (10) aufweist, wobei die zweite Sterilisationseinrichtung eine Strahlungserzeugungseinrichtung aufweist, welche eine Elektronenstrahlung erzeugt,
wobei
die Vorrichtung (1) eine Abschirmungseinrichtung (20) aufweist, welche die sich aus dieser sterilisierenden Strahlung ergebende Röntgenstrahlung wenigstens teilweise gegenüber der Umgebung abschirmt um einen Schutz des Bedieners und der Umgebung zu gewährleisten, wobei die Abschirmeinrichtung (20) ein Gehäuse (21) aufweist, welches die Transporteinrichtung (2) wenigstens teilweise umgibt, das Gehäuse (21) wenigstens eine Zuführöffnung (35) aufweist, um dem Gehäuse (21) die Behältnisse (10) zuzuführen und im Inneren des Gehäuses (21) Innenwandungen (23, 24, 25, 26, 28) angeordnet sind, welche dazu bestimmt sind und bewirken, dass die im Inneren entstehende Röntgenstrahlung wenigstens zweimal reflektiert wird, bevor sie zu der Zuführöffnung (35) gelangt, wobei diese Innenwandungen innerhalb des Gehäuses ein Labyrinth bilden, durch welches die Behältnisse (10) geführt werden,
**dadurch gekennzeichnet, dass**
die Abschirmvorrichtung (20) für einen geradlinigen Weg der Röntgenstrahlung nach außen eine Wand aus Blei in einer notwendigen Stärke von 5-6mm oder aus einem anderen Material mit einer äquivalenten Dicke aufweist.

2. Vorrichtung (1) nach Anspruch 1,
**dadurch gekennzeichnet, dass**
die Strahlungselemente (4) starr an der Transporteinrichtung (2) angeordnet sind.

3. Vorrichtung (1) nach wenigstens einem der vorangegangenen Ansprüche,
**dadurch gekennzeichnet, dass**
die zweite Sterilisationseinrichtung (12) stationär gegenüber dem Transportpfad der Behältnisse (10) angeordnet ist.

4. Vorrichtung (1) nach wenigstens einem der vorangegangenen Ansprüche,
**dadurch gekennzeichnet, dass**
die Vorrichtung (1) eine Dreheinrichtung aufweist, welche die Behältnisse wenigstens abschnittsweise um ihre Längsrichtung dreht

5. Verfahren zum Sterilisieren von Behältnissen (10), wobei die Behältnisse (10) entlang eines vorbestimmten Transportpfades (P) von einer Transporteinrichtung (2) transportiert und während dieses Transports mit einer Vielzahl von Sterilisationseinrichtungen (3) sterilisiert werden, wobei die Sterilisationseinrichtungen (3) jeweils sich stets parallel zur Längsrichtung der Behältnisse erstreckende Sterilisationselemente (4) aufweisen, welche durch die Mündung der Behältnisse (10) in den Innenraum der Behältnisse (10) gelangen, um eine Innenwandung der Behältnisse (10) durch Beaufschlagung mit einer Elektronenstrahlung zu sterilisieren, wobei auch eine Außenwandung der Behältnisse (10) wenigstens abschnittsweise mit Elektronenstrahlung beaufschlagt wird, um diese Außenwandung zu sterilisieren,
wobei
entstehende Strahlung durch ein als Abschirmeinrichtung dienendes Gehäuse (21) abgeschirmt wird, wobei das Gehäuse (21) wenigstens eine Zuführöffnung (35) aufweist, um den Gehäuse (21) die Behältnisse (10) zuzuführen und wobei im Inneren des Gehäuses (21) Innenwandungen (23, 24, 25, 26, 28) derart angeordnet werden, dass im Inneren entstehende sich aus der Elektronenstrahlung ergebende Röntgenstrahlung wenigstens zweimal reflektiert wird, bevor sie zu der Zuführöffnung gelangt, wobei diese Innenwandungen innerhalb des Gehäuses ein Labyrinth bilden, durch welches die Behältnisse (10) geführt werden,
**dadurch gekennzeichnet, dass**.
um einen Schutz des Bedieners und der Umgebung zu gewährleisen, als Abschirmvorrichtung (20) für einen geradlinigen Weg der Röntgenstrahlung nach außen eine Wand aus Blei in einer notwendigen Stärke von 5-6 mm oder aus einem anderen Material mit einer äquivalenten Dicke verwendet wird.

6. Verfahren zum Sterilisieren von Behältnissen nach Anspruch 5,
**dadurch gekennzeichnet, dass**
die Sterilisationselemente (4) in der Transportrichtung der Behältnisse (10) bewegt werden.

## Claims

1. Apparatus (1) for sterilising containers (10) with a transport device (2) which transports the containers (10) along a pre-specified transport path, with a multiplicity of sterilisation devices (3) for sterilising at least one region of the inner wall of the containers, wherein the first sterilisation devices each have a rod-like sterilisation element (4) which is formed as radiation element, the longitudinal direction (L) of which extends substantially perpendicular to the transport path of the containers and always parallel to the longitudinal direction of the containers to be sterilised of the containers and which by a relative movement of the container (10) in relation to the radiation element (4) can be introduced through a mouth (10a) of the containers (10) to be sterilised into the interior of the containers (10) to be sterilised, as well as a radiation generation device (6) forming a component of the first radiation device (3) which generates an electron radiation, wherein the apparatus (1) has at least one second sterilisation device (12) which is suitable and determined to sterilise at least one outer wall of the containers (10), wherein the second sterilisation device comprises a radiation generation device which generates an electron radiation,
wherein
the apparatus (1) comprises a shielding device (20), which at least partially shields the X-rays having been generated by said sterilising radiation with respect to the environment in order to allow for a protection of the operator and the environment, wherein the shielding device (20) comprises a housing (21) which at least partially surrounds the transport device (2), the housing (21) comprising at least one supply opening (35) in order to supply the containers (10) to the housing (21) and in the interior of the housing (21) inner walls (23, 24, 25, 26, 28) are arranged which are determined and effect that the X-rays being generated in the interior are reflected at least twice before reaching the supply opening (35), wherein these inner walls in the interior of the housing form a labyrinth, through which the containers (10) are guided,
**characterised in that**
the shielding device (20) for the linear way of the X-rays to the outside comprises a wall made of lead with a required thickness of 5-6 mm or of another material with an equivalent thickness.

2. Apparatus (1) according to claim 1,
**characterised in that**
the radiation elements (4) are rigidly arranged on the transport device (2).

3. Apparatus (1) according to at least one of the preceding claims, **characterised in that**
the second sterilisation device (12) is arranged stationary in relation to the transport path of the containers (10).

4. Apparatus (1) according to at least one of the preceding claims,
**characterised in that**
the apparatus (1) has a rotation device which rotates the containers at least in sections about their longitudinal direction.

5. Method for sterilising containers (10), wherein the containers (10) are transported along a predetermined transport path (P) by a transport device (2) and during this transport sterilised with a multiplicity of sterilisation devices (3), wherein the sterilisation devices (3) each have sterilisation elements (4) which always extend in parallel with regard to the longitudinal direction of the containers which arrive through the mouth of the containers (10) in the interior of the containers (10) in order to sterilise an inner wall of the containers (10) by the application of electron radiation, wherein also an outer wall of the containers (10) is at least partly exposed to electron radiation in order to sterilise this outer wall, wherein
the emerging radiation is shielded by a housing (21) serving as a shielding device, wherein the housing (21) has at least one supply opening (35) in order to supply the containers (10) to the housing (21) and wherein in the interior of the housing (21) inner walls (23, 24, 25, 26, 28) are arranged in such a way that the X-rays being generated in the interior and emerging from the electron radiation are reflected at least twice before reaching the supply opening, wherein these inner walls form a labyrinth in the interior of the housing, through which the containers (10) are guided,
**characterised in that**
in order to allow for a protection of the operator and the environment, as a shielding device (20) for the linear way of the X-rays to the outside a wall made of lead is used with a required thickness of 5-6 mm or of another material with an equivalent thickness.

6. Method for sterilising containers according to claim 5,
**characterised in that**
the sterilisation elements (4) are moved in the transport direction of the containers (10).

## Revendications

1. Installation (1) pour la stérilisation de récipients (10), avec un dispositif de transport (2) qui convoie les récipients (10) le long d'un chemin de transport défini, avec une pluralité de dispositifs de stérilisation (3) pour la stérilisation d'au moins une zone de la paroi intérieure des récipients, les premiers dispositifs de stérilisation présentant chacun un élément de stérilisation (4) en forme de barre, dont la direction longitudinale (L) s'étend sensiblement perpendiculairement au chemin de transport des récipients et toujours parallèlement au sens de la longueur des récipients à stériliser, et qui peut être introduit à l'intérieur des récipients (10) à stériliser par une bouche (10a) des récipients (10) à stériliser au moyen d'un déplacement relatif du récipient (10) par rapport à l'élément de rayonnement (4), ainsi qu'un dispositif générateur de rayonnement (6) faisant partie intégrante du premier dispositif de rayonnement (3) et qui génère un rayonnement électronique, ladite installation (1) comportant au moins un deuxième dispositif de stérilisation (12), adapté et prévu pour la stérilisation d'au moins une paroi extérieure des récipients (10), ledit deuxième dispositif de stérilisation présentant un dispositif générateur de rayonnement qui génère un rayonnement électronique,
ladite installation (1) comportant un dispositif d'isolation (20) qui isole au moins partiellement de l'environnement le rayonnement X résultant dudit rayonnement stérilisant, pour assurer une protection de l'opérateur et de l'environnement, le dispositif d'isolation (20) présentant un carter (21) entourant au moins partiellement le dispositif de transport (2), le carter (21) présentant au moins une ouverture d'admission (35) pour amener les récipients (10) dans le carter (21), et des parois intérieures (23, 24, 25, 26, 28) étant disposées à l'intérieur du carter (21), lesquelles sont prévues pour, et causent la réflexion, au moins deux fois, du rayonnement X généré à l'intérieur avant que celui-ci parvienne à l'ouverture d'admission (35), lesdites parois intérieures formant un labyrinthe à l'intérieur du carter, au travers duquel sont guidés les récipients (10),
**caractérisée en ce que**
le dispositif d'isolation (20) présente pour un trajet rectiligne du rayonnement X vers l'extérieur une paroi en plomb d'une épaisseur nécessaire de 5-6 mm, ou d'un autre matériau d'une épaisseur équivalente.

2. Installation (1) selon la revendication 1,
**caractérisée en ce que**
les éléments de rayonnement (4) sont disposés fixement sur le dispositif de transport (2).

3. Installation (1) selon au moins une des revendications précédentes,
**caractérisée en ce que**
le deuxième dispositif de stérilisation (12) est disposé de façon stationnaire par rapport au chemin de transport des récipients (10).

4. Installation (1) selon au moins une des revendications précédentes,
**caractérisée en ce que**
ladite installation (1) présente un dispositif de rotation qui tourne les récipients au moins en partie autour de leur direction longitudinale.

5. Procédé de stérilisation de récipients (10), lesdits récipients (10) étant convoyés par un dispositif de transport (2) le long d'un chemin de transport (P) prédéfini et étant stérilisés par une pluralité de dispositifs de stérilisation (3) pendant ce transport, lesdits dispositifs de stérilisation (3) présentant chacun des éléments de stérilisation (4) s'étendant toujours parallèlement au sens de la longueur des récipients, lesquels parviennent à l'intérieur des récipients (10) par la bouche des récipients (10) pour stériliser une paroi intérieure des récipients (10) par application d'un rayonnement électronique, un rayonnement électronique étant également appliqué au moins partiellement sur une paroi extérieure des récipients (10) pour stériliser ladite paroi extérieure,
un carter (21) servant de dispositif d'isolation isolant le rayonnement généré, ledit carter (21) présentant au moins une ouverture d'admission (35) pour amener les récipients (10) dans le carter (21), et des parois intérieures (23, 24, 25, 26, 28) étant disposées à l'intérieur du carter (21), de telle manière que le rayonnement X généré à l'intérieur et résultant du rayonnement électronique soit réfléchi au moins deux fois avant de parvenir à l'ouverture d'admission, lesdites parois intérieures formant un labyrinthe à l'intérieur du carter, au travers duquel sont guidés les récipients (10),
**caractérisé en ce que**
une paroi en plomb d'une épaisseur nécessaire de 5-6 mm, ou d'un autre matériau avec une épaisseur équivalente est utilisée en tant que dispositif d'isolation (20) pour un trajet rectiligne du rayonnement X vers l'extérieur, afin d'assurer une protection de l'opérateur et de l'environnement.

6. Procédé de stérilisation de récipients selon la revendication 5,
**caractérisé en ce que**
les éléments de stérilisation (4) sont déplacés dans la direction de transport des récipients (10).
